# EUROPEAN PATENT APPLICATION

(11) **EP 1 575 423 A1**
(43) Date of publication of application: **21.09.2005**
(21) Application number: 03809684.8
(22) Date of filing: 29.10.2003
(51) Int. Cl.: A61B 5/00, G01N 21/64

(54) **A LASER-INDUCED FLUORESCENCE METHOD FOR PRECANCEROUS LESION DIAGNOSIS AND AN ENDOSCOPE PRECANCEROUS LESION DIAGNOSIS APPARATUS THEREOF**

(30) Priority: 31.10.2002 CN 02137764
(71) Applicant: Shanghai Shengda Medical Health Stock Co., Ltd., Shanghai 201821 (CN)
(72) Inventor: ZENG, Kun, Shanghai 200233 (CN); YU, Zhenfen, Shanghai 200233 (CN); Huang, Zhouran, Shanghai 200233 (CN)
(74) Representative: Altenburg, Udo
(86) International application number: PCT/CN2003/000917
(87) International publication number: WO 2004/039254

(57) **Abstract**

A optical Biopsy method and an apparatus are used in the diagnosis of precancerous lesion for locating the place and determining the level of malignant tumor. The apparatus comprises light source (1, 10) a light channel system, an endoscope (21) and a circuit system. The light sources include an excited light (1) and a cold light source (10). The cold light source and the excited light in the light channel system go through the end of the light guide of the endoscope via optical fiber bundle and irradiate the tested living tissue (22). The white light image signal and the intrinsic fluorescence image signal reflected from the tested living tissue (22) are received by a weak fluorescence CCD (6) that tightly connects to the end of the endoscope (21) and then transmit to the circuit system via a signal wire (9) to produce the image in the display (17). The weak fluorescence signal reflected from the tested living tissue (22) is transmitted to the circuit system via the weak fluorescence fiber bundle (4) protruding from the forceps hole of the endoscope to produce the spectrum image (16).

## Description

### Field of Invention

This invention involves a method and its apparatus for the diagnosis of precancerous lesion. In particularly, this invent provides a optical Biopsy method for precancerous lesion diagnosis and a medical endoscope apparatus thereof.

### Background of Invention

At present, the incidence and mortality of malignant tumors are very high. The cause of this is that there is no great breakthrough in the diagnosis of the tumors, especially in the diagnosis of mucous membrane tumors. The diagnosis of these tumors still depends on the principle and method of morphology, i.e. the inspection of doctors, ultrasound, endoscopy, CT, MRI. However, these can only confirm if there is a space-occupying lesion, but can't tell if the lesion is benign or malignant. Morphologic diagnosis won't be able to differentiate early stage and moderate to severe atypical hyperplasia because the origin of the lesion is only minor biochemical changes in the mucous membrane which are not detectable by the ultrasound, endoscopy, CT or MRI.

Taking the gastric cancer as an example. It's learned from the developmental way of gastric cancer that the occurrence of metastasis through blood and lymph system from invasive cancer has taken place even in the early infiltrative stage, so once metastasis occurs, the difficulty to cure the disease will increase. This is a major cause of low cure rate and high mortality of gastric cancer.

The medical workers pay so much attention to the research and invention of the apparatus for the early diagnosis of malignant tumor. The patent of The Medical Instrument Company of Shanghai, Co. Ltd., "Apparatus using intrinsic fluorescence image and spectrum for the diagnosis of malignant tumor", described that the cold light source could reflect a signal after entering the endoscope through a bundle of optic fibers which may become the white light image by passing through an electronic system, the exciting light could also reflect a signal after entering the endoscope through a bundle of optic fibers which may display by passing through an electronic system. Therefore, the doctors can rapidly identify the location and nature of the tumor in multiple ways and so it can improve the sensitivity and specificity of the diagnosis of malignant tumors. However, this apparatus cannot diagnose the precancerous lesion. That is, a few years prior to the formation of the cancer, the apparatus can't tell whether the lesion will develope a benign one or a malignant one. Due to the uncertain diagnosis at early stage of the lesion, the patient could miss the possible treatments to prevent the lesion from developing to a malignant one, thus the difficulty to cure the disease increases and consequently, the incidence and mortality of cancer may rises.

### Summary of the invention

One objective of this invention is to design a optical Biopsy method to diagnose the precancerous lesion, which is able to locate the precancerous lesion (called as tested point or tested living tissue herein) in a comparatively higher accuracy, also be able to scan said tested living tissue rapidly and make a precise fluorescent spectrum curve and image.

Another objective of this invention is to design a conveniently and safely manipulated endoscope diagnostic apparatus for precancerous lesion, which is able to locate the precancerous lesion (called as tested point or tested living tissue herein) in a comparatively higher accuracy, also be able to scan said tested living tissue quickly and make a precise fluorescent spectrum curve and image.

The objectives of this invention are practiced as follows:
A optical Biopsy method for the Diagnosis of Precancerous Lesion,
Using light generated by a cold light source to irradiate the tested living tissue and the tested living tissue reflects the image signals of the tested irradiating white light;
Using a near violet light generated by excited light after being focused to irradiate the tested living tissue and the tested living tissue reflects the image signals of the tested intrinsic fluorescence;
Using a near violet light generated by excited light to irradiate the tested living tissue and the tested living tissue reflects the signals of tested weak fluorescence;
Combining the described tested white light image signals and the described tested intrinsic fluorescence image signals to produce the image of precancerous lesion site and consequently to grade the precancerous lesion;
Locating precancerous lesion and grading the precancerous lesion based on the described intrinsic fluorescence spectrum signals generated from the described tested weak fluorescence.

The described light channel system includes three channels. In first channel, the cold light source entry a port of the light guide of the endoscope after passing through an optical fiber bundle. The object port of the endoscope aims to, but not physically touches the tested living tissue. The cold light source irradiates the tested living tissue. The white light image signal reflected from the tested living tissue is received by a weak fluorescence CCD that tightly connects to the port of the endoscope and then transmit to the interface circuit via a signal wire; In second channel, the near violet light generated by the excited light passes through a focusing glass and reach the port of the endoscope via optical fiber bundle. The object port of the endoscope aims to, but not physically touches the tested living tissue. After the excited light irradiates the tested living tissue, the intrinsic fluorescence image signal reflected from the tested living tissue is received by a weak fluorescence CCD that tightly connects to the port of the endoscope and then transmit to the interface circuit via a signal wire. In third channel, the excited light as the second channel described above entry a port of the endoscope via optical fiber bundle, aims to and irradiates on the tested living tissue. The weak fluorescence signal reflected from the tested living tissue is transmitted to the OMA system via the weak fluorescence fiber bundle protruded from the forceps hole of the endoscope.

The described electronic system includes a weak light CCD that connects with the port of the endoscope tightly. The weak light CCD transmits the tested white light signals and the tested intrinsic fluorescence signals captured by it to the computer through an interface circuit, then the signals are sent to an image processor and an image display. The image is used to locate the precancerous lesion and to grade the precancerous lesion; the tested weak fluorescence signal transmitted from the weak fluorescence optical fibers goes through a rapid weak light spectrum analysis component - OMA system, from which intrinsic fluorescence spectrum signal is exported. The intrinsic fluorescence spectrum signal is then sent to the computer through a paralleled port. After that, it enters into a spectrum display by passing through a compressor. The spectrum is used to locate the precancerous lesion and to grade the precancerous lesion. Therefore, precancerous lesion can be located rapidly and graded exactly and promptly in multiple ways; the power switches of excited light and the cold light source are connected with a light transmitter, which is controlled by a pedal switch. The pedal switch is also connected with the paralleled port and the image processor.

The effect of this invention

When using the method and apparatus of this invention to diagnose the lesion of the tested tissue, the doctors can selectively observe the white light images, the intrinsic fluorescence images and the intrinsic fluorescence spectrum curves of the tested site according to their needs. The computer processes these images and spectrums curves so it's possible to identify the location of the precancerous lesion and the nature of the precancerous lesion clearly and rapidly in multiple ways, which improves the sensitivity and specificity of detection of the precancerous lesion. This allows the patients to receive relevant treatment as soon as possible according to their actual conditions and to reduce the probability of change from precancerous lesion to cancer, so the incidence and the mortality of cancer will be declined. This apparatus is of big social benefits and is suitable to be recommended among the hospitals.

In order to further explain the objectives, structural characters and effects of this invent described above, we will illustrate this invention in detail in combination with the attached figures.

### Brief Description of Drawings

Figure 1 shows the transformation of normal cells to cancerous cells and its relation to the environment of the host;
Figure 2 shows the early information from three stages of carcinomatous change - mutation, atypical hyperplasia, carcinoma *in situ* (CIS) ;
Figure 3 shows the scheme of principle of optical Biopsy method for the diagnosis of precancerous lesion;
Figure 4 shows a diagnostic report of intrinsic fluorescence spectrum;
Figure 5 is an embodiment for structure of the endoscope diagnosis apparatus for precancerous lesion of this invention;
Figure 6 is spectrum curve of grade 3 precancerous atypical hyperplasia displayed by spectrum method using the apparatus of this invention;
Figure 7 is the first spectrum curve of grade 2 precancerous atypical hyperplasia displayed by spectrum method using the apparatus of this invention;
Figure 8 is the second spectrum curve of grade 2 precancerous atypical hyperplasia displayed by spectrum method using the apparatus of this invention;
Figure 9 is spectrum curve of grade 1 precancerous atypical hyperplasia displayed by spectrum method using the apparatus of this invention.

### Detailed description of preferred embodiments

We will describe the structure and the usage of this invention in detail by reference to the attached figures of an embodiment as follows:

This invention is based on biochemistry and applies the spectrum technology to the spectrum detection of human body tissues. A diagnostic apparatus that uses optical biopsy (LIF, laser induced fluorescence), a worldwide-recognized precancerous detection technology, is invented. The diagnostic method and diagnostic criteria of LIF is known and approved internationally.

### Principle of related optical Biopsy technology:

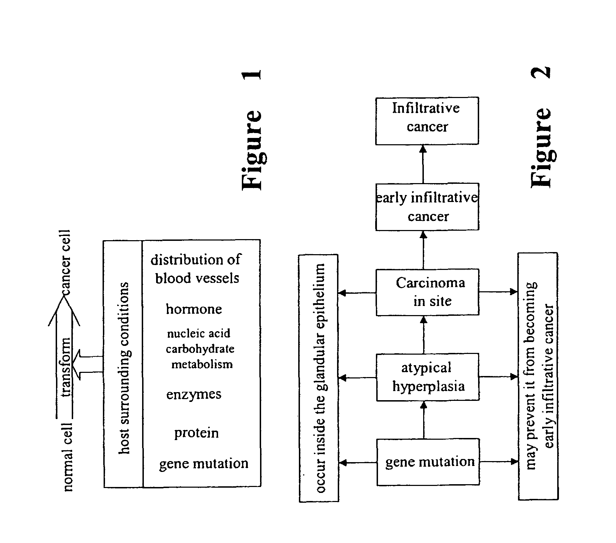

Firstly, see Figure 1, the basic biological character of cancerous cells is malignant proliferation, poor differentiation, infiltration and metastasis. These are well known morphologic changes. The viewpoint of biochemical change that normal cells transform cancerous cells is that, this transformation starts from the mutations of genes induced by carcinogenic factors, and then these gene mutations may result in intracellular change in proteins and enzyme expressing pattern due to abnormal gene expression. Enzyme is the catalyst of substance metabolism. So when activity of the enzyme changes drastically, the substance metabolism will definitely change accordingly. (Shown as Figure 1). Therefore, the change of nucleic acids, proteins and carbohydrates is closely related to the change of enzyme activity.

See Figure 2, epithelial cancerous lesion has the biggest impact on human beings. Most of malignant tumors are originated from epithelial tissues (including covering epithelium and glandular epithelium). For example, the gastric cancer is formed from the mutation, atypical hyperplasia and CIS of mucous glandular epithelium. The events during these three early stages of cancerous lesion are all limited inside the glandular epithelia mucous membrane (shown in figure 2). Since there is no real gastric cancer, there is no lymph metastasis. However, during these three stages, the nature of biochemistry inside the mucous membrane has already changed. The environment and conditions for abnormal proliferation and normal proliferation are different. So, without the special environment and condition provided by the host, abnormal proliferating cells are impossible to survive and develop. These events of early cancerous lesion are contained in the covering glandular epithelium and are never discovered and utilized by the scientists. If the characteristic information of precancerous lesion from a small area of the mucous membrane can be detected, then it is easily to prevent it from transferring to infiltrative cancer using a physical treatment by endoscope.

This invention, using the optical Biopsy technology, is able to find the apparent difference between precancerous information inside the mucous membrane and the information of normal mucous membrane. Its sensitivity, specificity and detection rate are 3-5 times higher than conventional diagnostic methods. This is ranked the fifth tumor diagnostic method after X-ray, ultrasound, CT and MRI described by an authoritative magazine - Light Lab.

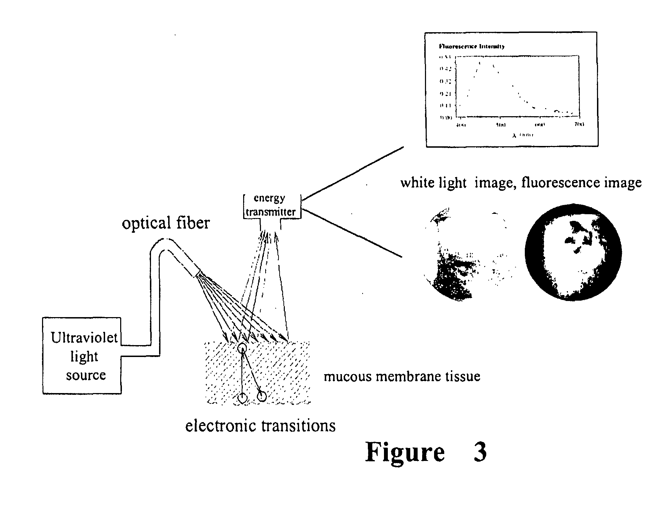

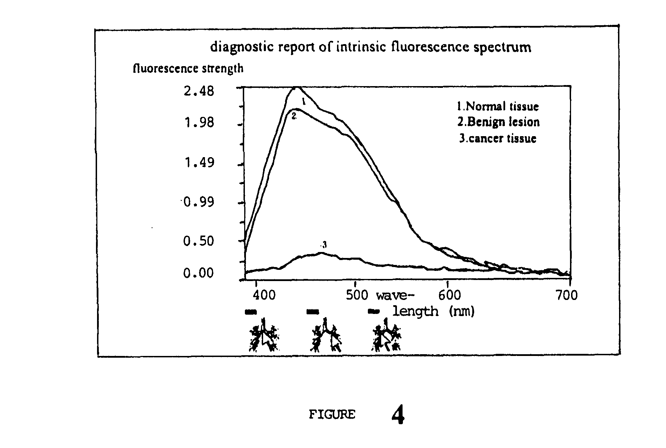

The science that studies the biology using electronic rules is called Quantum Biochemistry. It uses quantum mechanics as a tool for the biological research. That is, studying the sub-molecular biology from the electronic field. The molecular structure and the environment of all kinds of species (including biochemistry environment of the tumors) are different and they have the special spectrum rate of their own. When the light of a certain frequency irradiates on the species, under certain circumstances, the electrons may absorb the energy and make a transition to a higher energy level (excitation state). Most of the electrons are at a single excitation state. If the electrons make a direct transition from single excitation state to the basic state by radiation, they will emit corresponding light quantum to release the energy. This process is called fluorescence emission. We can see from this mechanism above that the generation of fluorescence is due to the change of quantum state in the molecular structure. Different molecular structure produces different wavelength of the fluorescence. Although the cancer tissue and its surrounding environment are not clear by now, but if there is light with enough energy to excite the cancerous tissue and normal tissue, the two different tissues can be identified because each of them can only absorb their own light quantum according to their ability of absorbing and the different light quantum absorbed by them will release different energy. These methods, by using the optical Biopsy method to diagnose the precancerous lesion, can be divided into two kinds and illustrated as Figure 3. One is spectrum method and the other is image method. These two methods both can identify whether the tested mucous membrane is normal, benign lesion or cancerous lesion. See figure 4, which is a diagnostic report of intrinsic fluorescence spectrum using spectrum method.

The method to differentially diagnose 'real' atypical hyperplasia by optical Biopsy:

Optical Biopsy technology has its unique advantage in the detection of epithelial atypical hyperplasia of mucous membrane. Its sensitivity and specificity are 3-5 times higher on average than conventional detections. Epithelial atypical hyperplasia is a pathological concept. According to its severity, atypical hyperplasia can be divided into three levels - mild, moderate and severe. However, atypical hyperplasia is a continuous developing procedure so it's difficult to be distinguished strictly. This kind of atypical hyperplasia is not only changes in histology and morphology, but also changes in biology (i.e. cell cancerous genes), which are common in terms of cancerous cells'.

Mild, moderate and severe atypical hyperplasia is strictly defined by pathology, which also indicates that atypical hyperplasia is similar in some way to cancer from the view of histology and molecular biology. But long-term follow-up results from clinical statistics demonstrated that mild atypical hyperplasia rarely transformed to cancer, while moderate and severe atypical hyperplasia often transformed to cancer clinically. There is a request for optical Biopsy technology to provide a borderline of mild, moderate and severe atypical hyperplasia, which is also a problem that requires an immediate resolution. After the inventors' practice and research, the spectrum method and image method have been established to differentiate mild, moderate and severe atypical hyperplasia as summarized in table 1 and table 2, respectively.

**Table 1**

| spectrum method to differentiate mild, moderate and severe atypical hyperplasia | | | |
|---|---|---|---|
| Type | 470nm | 680nm | 400nm |
| Normal tissue | 100% | No | No |
| Benign lesion | >70% | No | No |
| Severe atypical hyperplasia | <50% | Yes | Yes |
| Moderate atypical hyperplasia | <50% | Yes | No |
| Moderate atypical hyperplasia | <50% | No | Yes |
| Mild atypical hyperplasia | <50% | No | No |

**Table 2**

| Image method to differentiate mild, moderate and severe atypical hyperplasia | |
|---|---|
| Type | Color of tested tissue |
| Normal tissue | Blue and white |
| Benign lesion | Orange or orange red |
| Severe atypical hyperplasia | Violet red |
| Moderate atypical hyperplasia | Dark violet or dark red |
| Mild atypical hyperplasia | Dark |

The essential criteria of the diagnosis described above is whether the peak of fluorescence spectrum of tested mucous membrane tissue will appear at 680nm or 400nm. If these two peak appears, the tested tissue should be cancerous lesion, or moderate / severe atypical hyperplasia. Otherwise, although the peak at 470nm is <50% of normal, but no appearance of peak at 680nm or 400nm, the tissue is identified as mild atypical hyperplasia.

According to the theory described above, the apparatus of endoscope diagnosis for precancerous lesion is invented.

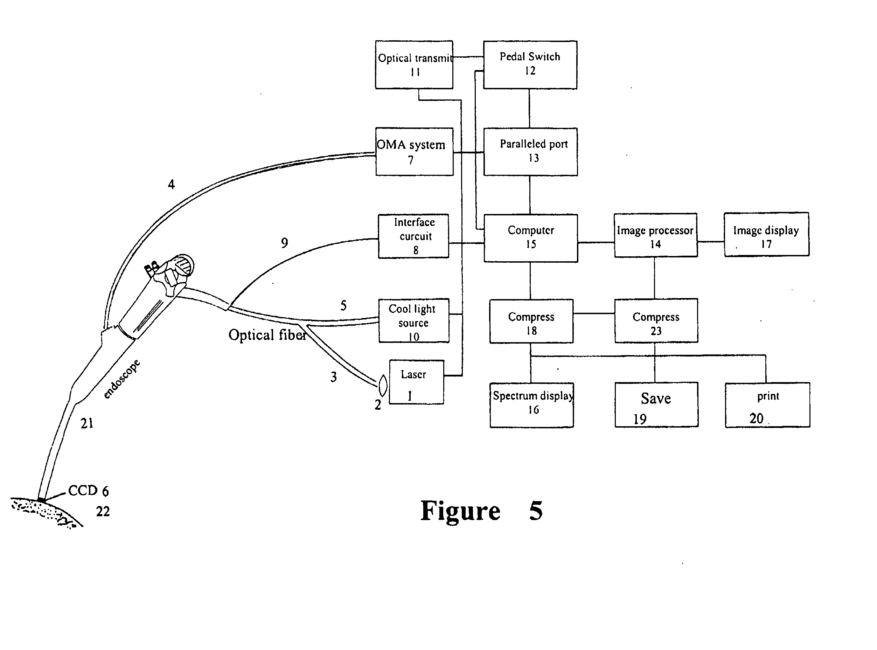

The structure of embodiment of this invention is shown in figure 5. It includes light source, optical system, endoscope and electric circuit system.

There are two light sources as described:

One is the excited light 1. Excited light 1 is a laser whose wavelength is 330nm-420nm. This can be excited light emitted by Nitrogen molecular laser or tripled frequency YAG laser or semiconductor laser or Hg light. At present invention, 337nm Nitrogen molecular laser is used. The other is cold light source 10, which is a halogen light at present invention.

The optical channel system as described above includes:

In first channel, the cold light source 10 entry a port of the guide light gun 21 of the endoscope via optical fiber bundle 5. The object port of the endoscope 21 aims to, but not physically touches the tested living tissue 22. The cold light source 10 irradiates the tested living tissue 22. The white light image signal reflected from the tested living tissue 22 is received by a weak fluorescence CCD that tightly connects to the port of the endoscope 21 and the signal received by weak light CCD transmit to the interface circuit 8 via a signal wire. In second channel, the near violet light generated by the excited light 1 passes through a focusing glass 2 and entry the port of the guide light gun of the endoscope 21 via optical fiber bundle 3. The object port of the endoscope 21 aims to, but not physically touches the tested living tissue 22. The excited light irradiates on the tested living tissue 22. The intrinsic fluorescence image signal reflected from the tested living tissue 22 is received by a weak fluorescence CCD that tightly connects to the end of the endoscope 21 and the signal received by weak light CCD transmit to the interface circuit 8 via a signal wire. In third channel, the excited light described above passes through the port of the endoscope 21 via optical fiber bundle 3 and aims to and directly irradiates on the tested living tissue 22. The tested weak fluorescence signal reflected from the tested living tissue 22 is transmitted to the OMA system via the weak fluorescence fiber bundle 4 protruding from the forceps hole of the endoscope 21 . The excited light optic fiber bundle 3 and cold light source optic fiber bundle 5 are included in a single bundle composed of low wasting quartz optical fibers.

The electronic system as described includes:

A weak light CCD 6 connects with a port of the endoscope 21 tightly (near the tested living tissue 22). The weak light CCD 6 transmits the tested white light signals and tested intrinsic fluorescence signals received by it to the computer 15 and an image processor 14 via a signal connection line 9 connecting with an interface circuit 8 and are further processed. The processed signals are sent to an image display 17 to display the tested white light image or tested intrinsic fluorescence image. At the same time, the signals from image processor 14 are compressed by a compressor 23 and are saved on disk 19 or printed out by a printer 20. The images can be used to locate the precancerous lesion and to grade the precancerous lesion; When the tested weak fluorescence signals transmitted from the weak fluorescence optical fibers 4 passes through a rapid weak light spectrum analysis component - OMA system 7, fluorescence signals as weak as <0.1 LUX reflected by the tested tissue may be detected. Fluorescence signals processed by OMA system are sent to the computer 15 for further processing through a paralleled port 13. After that, it passes through a compressor 18 for compressing and then enters into a spectrum display 16 to display the spectrum curves of intrinsic fluorescence and are saved in the disk 19 or printed out the spectrum curve of the intrinsic fluorescence by the printer 20. These spectrums can be used to locate the precancerous lesion and to grade the precancerous lesion. Therefore, precancerous lesion can be located and graded rapidly in multiple ways; the switches of excited light 1 and the cold light source 10 are connected with an optical transmitter 11, which is controlled by a pedal switch 12. Furthermore, the pedal switch 12 is also connected with the paralleled port 13 and the computer 15.

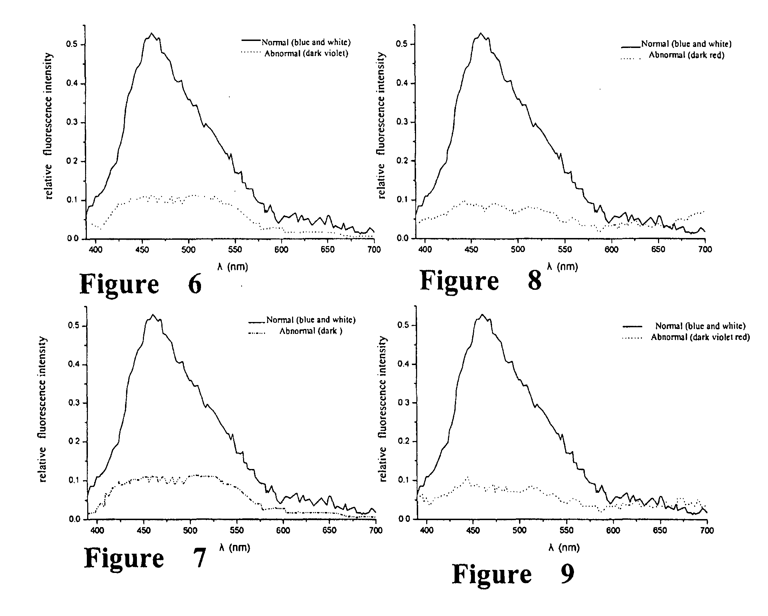

The method used by this diagnostic apparatus is to aim the tested living tissue 22 with the port of endoscope 21 but not touch it directly. Then using the pedal switch 12 to control the optical transmitter 11 to make the cold light source 10 work and using the pedal switch 12 to make the computer 15 work. Through the optical fibers 5 of the endoscope, cold light source 10 enters into the port of the endoscope 21 to irradiate on the tested living tissue 22. The reflected tested white light image is received by the weak light CCD 6, which connects to the endoscope 21 tightly. The weak light CCD records the signals and sends them to the computer 15 and image processor 14 where they are processed, via a signal transmitting line 9 connected with an interface circuit 8. The tested white light image will be displayed on the image display 17, and can be saved on the disk or be printed out. At the same time, the port of the endoscope 21 still aims to but is untouched with the tested living tissue 22. Using the pedal switch 12 to control the optical transmitter 11 to make the cold light source 10 or the excited light 1 work alternatively or individually. Also using the pedal switch 12 to make the computer 15 and the paralleled port 13 work. The excited light 1 enters into a bundle of optic fibers 3 after passing a focusing glass 2. Through the port of the endoscope 21 it irradiates on the tested living tissue 22. The reflected tested intrinsic fluorescence image signals are received by the weak light CCD 6 which connects to the port of the endoscope 21 tightly. The signals recorded by weak light CCD 6 are sent to the computer 15 and image processor 14 whereby to be processed via a signal transmitting line 9 connected with an interface circuit 8. The tested intrinsic fluorescence image and / or the tested white light image will be displayed on the image display 17. In comparison of the tested white light image and the tested intrinsic fluorescence image, if any suspected abnormal colorful area in the intrinsic fluorescence image is observed by the naked eyes, the excited light 1 via focusing glass 2 enters into the port of the endoscope 21, and then aims to and irradiates directly on the tested normal tissue and suspected tissue. The reflected tested weak fluorescence signals of normal and suspected tissues are transmitted by the weak fluorescence optic fibers 4 through the forceps hole of the endoscope 21 and enters a rapid weak light spectrum analysis component - OMA system 7. The rapid weak light spectrum analysis component - OMA system 7 detects the intrinsic fluorescence spectrum signals of normal tissue and the suspected tissue of the tested subject, respectively. Then the signals are displayed on the spectrum display 16 after passing through paralleled port 13 and processed by the computer 15. The tested normal tissue intrinsic fluorescence spectrum curve and / or the tested suspected tissue intrinsic fluorescence spectrum curve, as well as the ratio curve of these two spectrums, can be display on the spectrum display 16. The recorded spectrum curves (figure of ratio intensity E and wavelength nm) are shown in figure 6 - figure 9. The spectrum signals of tested living tissue described above which are sent to computer 15 to be processed via paralleled port 13 also can be saved on the disk and be printed out after being compressed by a compressor 18.

The peaks of the intrinsic fluorescence spectrum curve at three wavelengths (near 400nm, near 470nm and near 680nm) are the criteria for the diagnosis. In the figure, the curve with a higher peak (solid line) is the normal curve. The curve with a lower peak (dotted line) is the abnormal curve. If the tested tissue has a peak at 460-480nm, it can be identified as normal or abnormal according to the peak value. If the peak value of the tested normal area is defined 100%, then the peak value of the suspected area detected <50% of the normal peak value is abnormal. The following is, identified by the spectrum method of the apparatus of this invention, a table listing the different information of mild, moderate and severe atypical hyperplasia (grade 1, grade 2 and grade 3 atypical hyperplasia) which occurs before (5-6 years) the formation of cancerous lesion.

**Table 3**

| Different situation of grade 1, grade 2 and grade 3 atypical hyperplasia which occurs before the formation of cancerous lesion by using the spectrum method of the apparatus of this invention. | | | | | | |
|---|---|---|---|---|---|---|
| Type | 460-480 | 390-420 | 670-690 | Color of fluorescence | Tendency of the lesion | Figure |
| Grade 1 | <50% | No peak | No peak | Dark | Not susceptible to cancer | 6 |
| Grade 2 | <50% | Peak | No peak | Dark violet | Possibly to cancer | 7 |
| Grade 2 | <50% | No peak | Peak | Dark red | Possibly to cancer | 8 |
| Grade 3 | <50% | Peak | Peak | Dark violet red | Susceptible to cancer | 9 |

The suspected area seen on the intrinsic fluorescence image is precancerous lesion.

The diagnosis from multiple ways described above improves the sensitivity and specificity of detection of precancerous tissue. This allows the patients to receive relevant treatment as soon as possible according to their actual conditions and to reduce the incidence of transform from precancerous lesion to cancer.

It should be understood by those skilled in the art that the examples described herein are provided for the purpose of illustration and are not intended as limitations on the scope of the invention. Certain changes and modifications of examples described above without departing from the spirit of this invent intend to be encompassed in the scope of the claims.

## Claims

1. A optical Biopsy method for the diagnosis of precancerous lesion comprising
• A light generated by a cold light source is used to irradiate the tested living tissue from which the tested white light image signals are reflected;
• A focused near violet light generated by excited light is used to irradiate the tested living tissue from which the tested intrinsic fluorescence image signals are reflected;
• A focused near violet light generated by excited light is used to irradiate the tested living tissue from which the tested weak fluorescence signals are reflected;
• Said tested white light image signals and said tested intrinsic fluorescence image signals are combined to produce a image of the precancerous lesion site for grading the precancerous lesion;
• An intrinsic fluorescence spectrum signals are generated from said tested weak fluorescence by which the precancerous lesion can be located and graded.

2. The optical Biopsy method of claims 1, comprising the step of detecting the wave shape of said intrinsic fluorescence spectrum signals at 470nm, 680nm and 400nm when identifying mild, moderate and severe atypical hyperplasia,
• If the peak value at 470nm of the tested tissue is more than 70% of that of normal tissue, and there are no peaks at 680nm and 400nm, the lesion is thought to be benign;
• If the peak value at 470nm of the tested tissue is 50% less than that of normal tissue, and there are peaks at 680nm and 400nm, the lesion is thought to be severe atypical hyperplasia;
• If the peak value at 470nm of the tested tissue is 50% less than that of normal tissue, and there is only one peak at 680nm or at 400nm, the lesion is thought to be moderate atypical hyperplasia;
• If the peak value at 470nm of the tested tissue is 50% less than that of normal tissue, and there is no peak at either 680nm or 400nm, the lesion is thought to be mild atypical hyperplasia.

3. The optical Biopsy method of claims 1, wherein the color shown by the image of said precancerous lesion is as follows during identifying mild, moderate and severe atypical hyperplasia,
• blue and white for the normal tissue;
• orange or orange red for benign lesion;
• violet red for severe atypical hyperplasia;
• dark violet or dark red for moderate atypical hyperplasia;
• dark colors for mild atypical hyperplasia.

4. The optical Biopsy method of claims 1, comprising the step of detecting the wave shape of said intrinsic fluorescence spectrum signals at 460nm-480nm, 390-410nm and 670nm-690nmits when identifying grade 1, grade 2 and grade 3 atypical hyperplasia,
• If the peak value at 460nm-480nm is 50% less than that of normal tissue, and there are no peaks at 390nm-420nm and 670nm-690nm, the lesion is thought to be grade 1 atypical hyperplasia, the fluorescence color shown by said precancerous lesion image is dark color, the lesion will susceptible not develop to cancer;
• If the peak value at 460nm-480nm is 50% less than that of normal tissue, and there is only one peak at 390nm-420nm or 670nm-690nm, the lesion is thought to be grade 2 atypical hyperplasia, the fluorescence color shown by said precancerous lesion image is dark violet or dark red, the lesion will possibly develop to cancer;
• If the peak value at 460nm-480nm is 50% less than that of normal tissue, and there are peaks at both 390nm-420nm and 670nm-690nm, the lesion is thought to be grade 3 atypical hyperplasia, the fluorescence color shown by said precancerous lesion image is dark violet red, the lesion will susceptible develop to cancer.

5. An apparatus of endoscope diagnosis of precancerous lesion using optical Biopsy method of claims 1, said apparatus comprises light source, optical channel system, endoscope and electronic system, wherein said light source includes an excited light and a cold light source, its characters are,
• said optical channel system includes: in first channel, the cold light source entry a port of the light guide of the endoscope by passing through an optical fiber bundle, the object port of the endoscope aims to, but not physically touches the tested living tissue, the cold light source irradiates the tested living tissue, the white light image signal reflected from the tested living tissue is received by a weak fluorescence CCD that tightly connects to the port of the endoscope and then transmit to the interface circuit via a signal wire; in second channel, the near violet light generated by the excited light passes through a focusing glass and reach the port of the endoscope via optical fiber bundle, the object port of the endoscope aims to, but not physically touches the tested living tissue, after the excited light irradiates the tested living tissue, the intrinsic fluorescence image signal reflected from the tested living tissue is received by a weak fluorescence CCD that tightly connects to the port of the endoscope and then transmit to the interface circuit via a signal wire; in third channel, the excited light as the second channel described above entry a port of the endoscope via optical fiber bundle, aims to and irradiates on the tested living tissue, the weak fluorescence signal reflected from the tested living tissue is transmitted to the OMA system via the weak fluorescence fiber bundle protruded from the forceps hole of the endoscope;
• said electronic system includes a weak light CCD which connects with the port of the endoscope tightly, the weak light CCD transmits the tested white light signals and the tested intrinsic fluorescence signals captured by it to the computer through an interface circuit, then the signals are sent to an image processor and an image display, the image is used to locate the precancerous lesion and to grade the precancerous lesion; the tested weak fluorescence signal transmitted from the weak fluorescence optical fibers goes through a rapid weak light spectrum analysis component - OMA system, from which intrinsic fluorescence spectrum signal is exported, the intrinsic fluorescence spectrum signal is then sent to the computer through a paralleled port, after that, it enters into a spectrum display by passing through a compressor, the spectrum is used to locate the precancerous lesion and to grade the precancerous lesion, therefore, precancerous lesion can be located rapidly and graded exactly and promptly in multiple ways; the power switches of excited light and the cold light source are connected with a light transmitter which is controlled by a pedal switch, the pedal switch is also connected with the paralleled port and the image processor.

6. The apparatus of endoscope diagnosis for precancerous lesion of claims 5, its character is that the wavelength of said excited light is 330nm-420nm.

7. The apparatus of endoscope diagnosis for precancerous lesion of claims 5, its character is that said excited light optic fiber bundle and cold light source optic fiber bundle are included in a single bundle composed of multiple low wasting quartz optical fibers.

8. The apparatus of endoscope diagnosis for precancerous lesion of claims 5, its character is that said image signals of the tested living tissue from the image processor are sent to an image display.

9. The apparatus of endoscope diagnosis for precancerous lesion of claims 5, its character is that said image signals of the tested living tissue from the image processor are saved to disk or printed out by a printer after being compressed by a compressor.

10. The apparatus of endoscope diagnosis for precancerous lesion of claims 5, its character is that the spectrum signals of the tested living tissue sent to computer by the paralleled port and processed by the computer are saved to disk or printed out by a printer after being compressed by a compressor.
